# EUROPÄISCHE PATENTSCHRIFT

(11) **EP 0 671 150 B1**
(45) Veröffentlichungstag und Bekanntmachung des Hinweises auf die Patenterteilung: **21.11.2001**
(21) Anmeldenummer: 95250029.6
(22) Anmeldetag: 07.02.1995
(51) Int. Cl.: A61B 17/34

(54) **Medizinisches Gerät zur Punktion organischen Gewebes**
Medical device for puncturing organic tissue
Dispositif médical pour ponction de tissu organique

(30) Priorität: 09.02.1994 DE 4404798
(43) Veröffentlichungstag der Anmeldung: 13.09.1995
(73) Patentinhaber: Otten, Gert, Prof., Dr., 27619 Schiffdorf (DE)
(72) Erfinder: Otten, Gert, Prof. Dr. med., D-27619 Schiffdorf (DE); Lindeke, Carsten, D-12459 Berlin (DE)
(74) Vertreter: Porth, Hans-Joachim

(56) Entgegenhaltungen:
- EP-A- 0 445 378
- DE-A- 2 657 053
- DE-A- 3 917 051
- FR-A- 1 035 923
- US-A- 1 147 408

## Beschreibung

Die Erfindung betrifft ein medizinisches Gerät nach dem Oberbegriff des Patentanspruchs 1.

Es sind bereits derartige Geräte bekannt. Die Punktion mittels dieser Geräte erfolgt, indem der Schaft des Geräts auf das Gewebe aufgesetzt, die längs und innerhalb des Schaftes längsverschiebliche Kanüle eingestochen, letztere nach der Probeentnahme aus dem Gewebe herausgezogen und sodann das Gerät vom Organ abgenommen wird. Sehr häufig bilden sich insbesondere sofort nach der Abnahme des Geräts Blutungen im Bereich des Einstichs. Diese erschweren zum einen die Heilung bzw. das Verschließen der Einstichstelle. Sie können zum anderen unter Umständen sogar weitere medizinische Eingriffe erfordern.

Ein derartiges medizinisches Gerät ist aus der DE-A-39 17 051 bekannt. Diese offenbart. daß eine hämostatische gelatinöse Hülse als Teil einer äußeren Kanüle über dem distalen Teil der inneren Kanüle einer Biopsienadel angeordnet ist. Zur Minimierung einer infolge der Gewebeentnahme auftretenden Blutung wird die gelatinöse Hülse mit einer Einstellvorrichtung genau an der Stelle abgesetzt, wo die Biopsieprobe entnommen wurde. Dabei wird die Hülse, nachdem die Biopsienadel mit der Gewebeprobe herausgezogen wurde, an der Biopsiestelle zurückgelassen. Die Hülse minimiert somit bei gleichzeitiger Auflösung im Körper die Blutung. Dieses Gerät ist wegen der Verwendung dreier Kanülen, nämlich einer Schneidkanüle in einer Zwischenkanüle und diese wiederum in einer äußeren Hülse angeordnet, kompliziert aufgebaut. Hinzu kommt, daß die äußere Hülse zweigeteilt ist und deren gelatinöser Teil für jede Gewebeentnahme erneuert werden muß.

Der Erfindung liegt die Aufgabe zugrunde, ein medizinisches Gerät der eingangs erwähnten Gattung zu schaffen, welches eine problemlose Punktion mit einfachen Mitteln ermöglicht und eine Verringerung der Belastungen des Patienten wie auch der Risiken des Eingriffs gestattet.

Erfindungsgemäß wird die Aufgabe dadurch gelöst, daß der Schaft einerseits, nämlich auf der Seite, auf der die Kanüle zur Punktion austritt, einen Aufsatzkopf mit einer Anlagefläche für das Gewebe aufweist, die koaxial zu einer Führungsbohrung für die Kanüle konisch ausgebildet ist. Ein Teil des Aufsatzkopfes ist zudem durch eine Elektrode zum Koagulieren des Gewebes im Bereich der Einstichstelle der Kanüle gebildet. Beim Anpressen des Aufsatzkopfes auf das Gewebe wird somit zum einen im Bereich der Einstichstelle der Kanüle gezielt zumindest teilweise die Durchblutung gemindert und zum anderen erfolgt das Koagulieren des Gewebes bei noch angepreßtem Aufsatzkopf, wodurch Blutungen infolge der Gewebeentnahme weitestgehend ausgeschlossen werden können. Wegen der konischen Ausbildung der Anlagefläche des Aufsatzkopfes ist die von der Anlagefläche auf das Gewebe ausgeübte Flächenpressung unmittelbar an der Einstichstelle groß und nimmt nach außen ab, so daß die Gewebedurchblutung an der Einstichstelle am stärksten und nach außen abnehmend gemindert ist. Die Blutgefäße werden zwangsläufig infolge der Anpressung des Aufsatzkopfes angepaßt an deren Verletzung abgedichtet. Vorteilhaft ist dabei auch, daß das Koagulieren zwangsläufig dort geschieht, wo der Einstich zuvor tatsächlich erfolgte. Das Verschließen der Einstichstelle ist abgeschlossen, bevor die Minderung der Durchblutung infolge des Anpressens des Aufsatzkopfes aufgehoben wird. Die durch Koagulieren behandelte Gewebefläche kann auf das unbedingt erforderliche Maß beschränkt werden.

Zweckmäßigerweise ist die Anlagefläche zumindest teilweise kegel-, pyramiden- und/oder kugelförmig ausgebildet, was zum einen die von der Einstichstelle nach außen abnehmende Pressung des Gewebes in einfacher Weise ermöglicht und zum anderen ein sehr präzises und gezieltes Plazieren des Aufsatzkopfes und somit der Kanüle gestattet.

Weitere Merkmale der Erfindung und deren Vorteile ergeben sich aus den übrigen Ansprüchen und der Beschreibung.

Die Erfindung soll nachfolgend an einem Ausführungsbeispiel näher erläutert werden. In der zugehörigen Zeichnung zeigen:

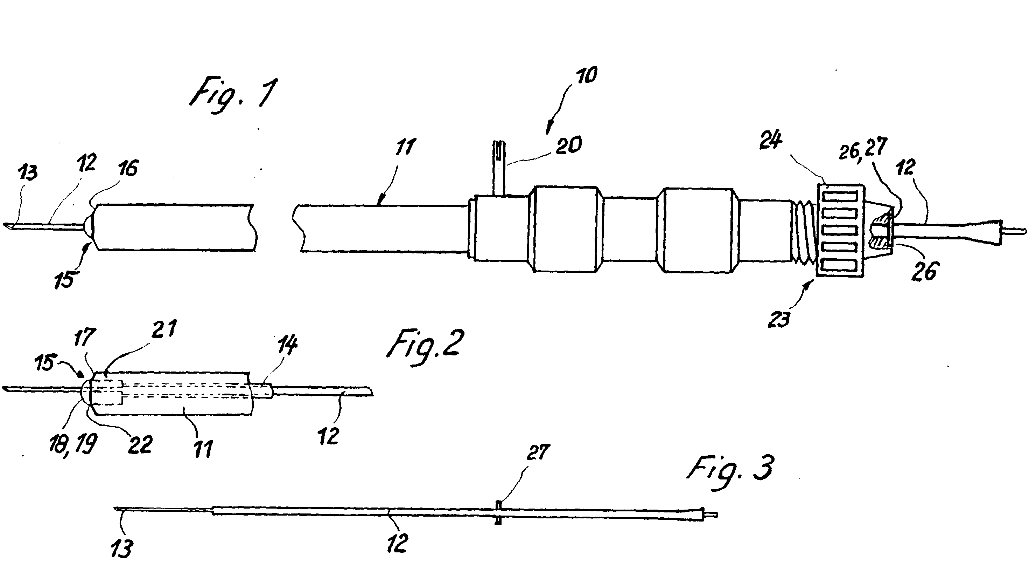

- Fig. 1: eine Seitenansicht des medizinischen Geräts, schematisch dargestellt,
- Fig. 2: die Ansicht nach Fig. 1 teilweise und teilweise geschnitten dargestellt und
- Fig. 3: die Seitenansicht einer Kanüle schematisch dargestellt.

Das Gerät 10 zur Punktion eines Organs bzw. organischen Gewebes ist mit einem Schaft 11 versehen. Auf der Seite des Schaftes 11, auf der zur Punktion eine Kanüle 12 mit einer Spitze 13 aus einer Führungsbohrung 14 des Schafts 11 austritt, endet der Schaft 11 in einen Aufsatzkopf 15. Mit diesem wird das Gerät 10 auf das zu punktierende Organ aufgesetzt und gegen das Gewebe gepreßt, während die Spitze 13 der Kanüle 12 noch in der Führungsbohrung 14 des Schafts 11 angeordnet ist. Durch das Aufpressen des Aufsatzkopfes 15 erfolgt eine Minderung der Durchblutung des Gewebes im Bereich der vorgesehenen Einstichstelle. Die Durchblutung ist wegen der geometrischen Gestalt einer Anlagefläche 16 des Aufsatzkopfes 15 ungleichmäßig beeinflußt und am stärksten unmittelbar an der Einstichstelle der Kanüle 12. Dazu ist die Anlagefläche 16 koaxial zu der Führungsbohrung 14 für die Kanüle 12 konisch, d.h. kegel-, pyramiden- und/oder kugelabschnittförmig ausgebildet.

Die Anlagefläche 16 hat in einem peripheren äußeren Bereich 17 einen kegelförmigen Verlauf, so daß in diesem Bereich 17 der Aufsatzkopf 15 ein Kegelstumpf ist. In einem zentralen Bereich 18 dagegen ist die Anlagefläche 16 kugelabschnittförmig ausgebildet, so daß dieser Bereich 18 des Aufsatzkopfes 15 etwa eine Halbkugel ist. Die Kanüle 12 tritt im Zenit des zentralen Bereichs 18 aus der Führungsbohrung 14 und damit dem Aufsatzkopf 15 aus.

Der zentrale Bereich 18 der Anlagefläche 16 bzw. ein entsprechender Teil des Aufsatzkopfes 15 ist durch eine Elektrode 19 zum Koagulieren des Gewebes im Bereich der Einstichstelle der Kanüle 12 gebildet. Die Elektrode 19 besteht aus einem sehr gut wärmeleitfähigem vorzugsweise metallenem Werkstoff. Das Koagulieren erfolgt mittels Hochfrequenzstrom, der über einen Anschluß 20 zur Elektrode 19, über diese zum anliegenden Gewebe und schließlich über den Körper des Patienten zu einer am Körper des Patienten mit Abstand anliegenden Anode geleitet wird. Der Schaft 11 kann dazu einen metallenen Innenmantel 21 aufweisen, der zur Leitung des Hochfrequenzstroms zur Elektrode 19 dient. Dieser Innenmantel 21 ist elektrisch gegenüber dem übrigen Schaft 11 isoliert.

Der übrige Schaft 11 und insbesondere der Teil, der den peripheren Bereich 17 der Anlagefläche 16 umfaßt. besteht aus einem elektrisch nichtleitendem und extrem gut wärme isolierendem Material, beispielsweise einem Kunststoff. Hierdurch ist sichergestellt, daß das Koagulieren allein durch den zentralen Bereich 18 der durch die Elektrode 19 gebildeten Anlagefläche 16 und nicht auch im peripheren Bereich 17 erfolgt. Das zu koagulierende Gewebe kann somit auf das zum Verschließen der Einstichstelle unbedingt erforderliche Maß beschränkt werden. Diese Behandlung erstreckt sich demnach nicht auf den gesamten Abschnitt mit geminderter Durchblutung.

Wegen der unterschiedlichen Gestalt des peripheren und zentralen Bereichs 17, 18 und eines gewissermaßen stufenförmigen Übergangs 22 zwischen diesen, wird eine besonders starke Anpressung des Aufsatzkopfes 15 unmittelbar um die Einstichstelle herum erreicht. Die damit einhergehende stärkste Minderung der Durchblutung erfolgt somit unmittelbar an der Einstichstelle, was zum einen den Einstich selbst wie auch das spätere Koagulieren begünstigt.

Mit dem Gerät 10 kann eine zu punktierende Stelle sehr exakt und gezielt vorfixiert, die eigentliche Punktion bei definiert geminderter Beeinflussung des Gewebes unmittelbar um die Einstichstelle herum durchgeführt und noch unter diesen Bedingungen nach dem Herausziehen der Kanüle 12 aus dem Gewebe die Einstichstelle bzw. die dort befindlichen verletzten Blutgefäße verschlossen werden. Das Abnehmen des Aufsatzkopfes 15 erfolgt also erst, nachdem der Einstich koaguliert ist. Die Ausbildung von Blutungen infolge der nunmehr wieder ungeminderten Durchblutung ist ausgeschlossen.

Schließlich weist der Schaft 11 an einem dem Aufsatzkopf 15 gegenüberliegenden Ende eine Stelleinrichtung 23 zur Fixierung der Einstichtiefe der Kanüle 12 auf. Diese wird durch eine Stellmutter 24 gebildet, die über korrespondierend ausgebildete Gewinde des Schaftes 11 und der Stellmutter 24 zum Schaft 11 dreh- und damit auch axialverschieblich ist. Auf der Seite der Stellmutter 24, die dem Schaft 11 abgewandt ist, endet eine Führungsbohrung der Stellmutter 24 für die Aufnahme der Kanüle 12 in einer Aufnahme 26. Die Kanüle 12 ist in dieser Führungsbohrung in Richtung des Aufsatzkpfes 15 axialverschieblich, bis ein Anschlag 27 der Kanüle 12 in der Aufnahme 26 der Führungsbohrung zur Anlage kommt. Die mögliche Einstichtiefe der Kanüle 12 ist demnach groß. sofern die Stellmutter 24 weit auf den Schaft 11 aufgeschraubt ist und umgekehrt. Hierdurch ist eine feine gestufte, sehr exakte und vor allem einfachste Einstellung der Einstichtiefe der Kanüle 12 möglich.

Der Anschlag 27 kann als quer zur Kanüle 12 verlaufender Stift, kegelförmige Verdickung oder dgl. ausgebildet sein, wobei die Aufnahme 26 und der Anschlag 27 stets eine korrespondierende Gestalt aufweisen.

### Bezugszeichenaufstellung

- 10: Gerät
- 11: Schaft
- 12: Kanüle
- 13: Spitze
- 14: Führungsbohrung
- 15: Aufsatzkopf
- 16: Anlagefläche
- 17: Bereich
- 18: Bereich
- 19: Elektrode
- 20: Anschluß
- 21: Innenmantel
- 22: Übergang
- 23: Stelleinrichtung
- 24: Stellmutter
- 26: Aufnahme
- 27: Anschlag

## Patentansprüche

1. Medizinisches Gerät (10) zur Punktion organischen Gewebes, mit einem Schaft (11) und einer zum Einstechen und Entnehmen einer Gewebeprobe zum Schaft (11) axialverschieblichen Kanüle (12), **dadurch gekennzeichnet, daß** der Schaft (11) auf der Seite, auf der die Kanüle (12) zur Punktion austritt, einen Aufsatzkopf (15) mit einer Anlagefläche (16) für das Gewebe aufweist, die koaxial zu einer Führungsbohrung (14) für die Kanüle (12) konisch ausgebildet ist, derart. daß die Beeinflussung der Durchblutung von der Mitte zum Rand des Einstichbereichs abnimmt und ein Teil des Aufsatzkopfes durch (15) eine Elektrode (19) zum Koagulieren des Gewebes im Bereich des Einstichs der Kanüle (12) bei noch angepreßtem Aufsatzkopf (15) gebildet ist.

2. Medizinisches Gerät nach Anspruch 1, **dadurch gekennzeichnet, daß** die Anlagefläche (16) zumindest teilweise kegel-, pyramiden- und/oder kugelförmig ausgebildet ist.

3. Medizinisches Gerät nach Anspruch 1 und 2, **dadurch gekennzeichnet, daß** die Anlagefläche (16) in einem peripheren und einem zentralen Bereich (17; 18) unterschiedlich ausgebildet ist.

4. Medizinisches Gerät nach Anspruch 3, **dadurch gekennzeichnet, daß** die Anlagefläche (16) im peripheren Bereich (17) kegelförmig und im zentralen Bereich kugelabschnittförmig ausgebildet ist.

5. Medizinisches Gerät nach einem oder mehreren der Ansprüche 3 und 4, **dadurch gekennzeichnet, daß** wenigstens der zentrale Bereich (18) der Anlagefläche (16) durch die Elektrode (19) gebildet ist.

6. Medizinisches Gerät nach einem oder mehreren der Ansprüche 1 bis 5, **dadurch gekennzeichnet, daß** die Elektrode (19) aus Werkstoff mit hoher Wärmeleitfähigkeit besteht und zum Koagulieren über die Elektrode (19) Hochfrequenzstrom auf das Gewebe und eine diesem benachbarte Anode leitbar ist.

7. Medizinisches Gerät nach einem oder mehreren der Ansprüche 3 und 4, **dadurch gekennzeichnet, daß** der periphere Bereich (17) der Anlagefläche (16) durch eine Stirnfläche des Schafts (11) gebildet ist und der Schaft (11) wenigstens im Bereich des Aufsatzkopfes (15) aus elektrisch nicht leitendem Werkstoff mit hohen Temperaturisoliereigenschaften besteht.

## Claims

1. Medical device (10) for puncturing organic tissue with a shaft (11) and a canula (12) that can be shifted axially to the shall (11) for puncturing and removing tissue samples, **characterized by** the shaft (11) having an extended head (15) on the side on which the canula (12) extends for the puncture, with a surface (16) for the tissue. The surface is conically shaped coaxial to a guide (14) for the canula (12) in such a manner that influence of the circulation decreases from the middle to the edge of the puncture area and a portion of the head (15) is formed by an electrode (19) for coagulation of the tissue in the area of the puncture of the canula (12) from the pressure of the head (15).

2. Medical device according to claim 1, **characterized by** the surface (16) at least partially cone, pyramid and/or spherical-shaped.

3. the surface (16) being differently shaped in a peripheral and a central area (17; 18).

4. Medical device according to claim 3, **characterized by** the surface (16) in the peripheral area (17) being cone-shaped and semi-spherical in the central area.

5. Medical device according to one or more of claims 3 and 4, **characterized by** at least the central area (18) of the surface (16) being formed by the electrode (19).

6. Medical device according to one or more of claims 3 and 4, **characterized by** the electrode (19) being made of a substance with a high heat-conductivity and high-frequency current being conducted through the electrode (19) to the tissue and a neighboring anode, for the purpose of coagulation.

7. Medical device according to one or more of claims 3 and 4, **characterized by** the peripheral area (17) of the surface (16) being formed by the end surface of the shaft (11) and the shaft (11) is made of a material that does not conduct electricity with a high-temperature insulation property, at least in the area of the head (15).

## Revendications

1. Instrument médical (10) de ponction de tissu organique muni d'une tige (11) et d'une canule (12) susceptible de se déplacer axialement par rapport à la tige (11) afin de piquer et de prélever un échantillon de tissu, **caractérisé par le fait que** la tige (11) présente, du côté duquel la canule (12) de ponction émerge, un adaptateur (15) muni d'une surface d'appui (16) pour le tissu dont la configuration- conique est coaxiale par rapport à un perçage de guidage (14) pour la canule (12), de telle façon que l'influence de l'irrigation. sanguine décroît du milieu vers le bord de la zone piquée et une partie de l'adaptateur (15) est formée par -une électrode (19) destinée à coaguler le tissu dans la zone piquée par la canule (12) lorsque l'adaptateur (15) est encore pressé.

2. Instrument médical selon la revendication 1, **caractérisé par le fait que** la surface d'appui (16) a une configuration au moins en partie conique, pyramidale et/ou sphérique.

3. Instrument médical selon la revendication 1 et 2, **caractérisé par le fait que** la surface d'appui (16) a une configuration différente dans une -zone périphérique et une zone centrale (17;18).

4. Instrument médical selon la revendication 3, **caractérisé par le fait que** la surface d'appui (16) a une configuration conique dans la zone périphérique (17) et une section de forme sphérique dans la zone centrale.

5. Instrument médical selon l'une ou plusieurs des revendications 3 et 4, **caractérisé par le fait qu**'au moins la zone centrale (18) de la surface d'appui (16) est formée par une électrode (19).

6. Instrument médical selon l'une ou plusieurs des revendications 1 à 5, **caractérisé par le fait que** l'électrode (19) se compose d'une matière première présentant une conductibilité thermique élevée et qu'en vue de la coagulation, un courant haute fréquence est susceptible d'être conduit par l'intermédiaire de l'électrode (19) sur le tissu et sur une anode adjacente à celui-ci.

7. Instrument médical selon l'une ou plusieurs des revendications 3 et 4, **caractérisé par le fait que** la zone périphérique (17) de la surface d'appui (16) est formée par une frontale de la tige (11) et que la tige (11) se compose au moins dans la zone de l'adaptateur (15) d'une. matière première non conductrice de courant électrique présentant des propriétés calorifuges élevées.
